# EUROPÄISCHE PATENTSCHRIFT

(11) **EP 1 754 438 B1**
(45) Veröffentlichungstag und Bekanntmachung des Hinweises auf die Patenterteilung: **27.05.2015**
(21) Anmeldenummer: 06017240.0
(22) Anmeldetag: 18.08.2006
(51) Int. Cl.: A61B 1/018

(54) **Endoskop, insbesondere Duodendoskop für die Mutter-Baby-Cholangioskopie**
Endoscope, in particular duodendoscope for mother-baby- cholangioscopy
Endoscope, notamment duodendoscope pour mère-enfant-cholangioscopy

(30) Priorität: 19.08.2005 DE 102005039601
(43) Veröffentlichungstag der Anmeldung: 21.02.2007
(73) Patentinhaber: Karl Storz GmbH & Co. KG, 78532 Tuttlingen (DE)
(72) Erfinder: Frimberger, Eckart, Prof. Dr. med., 80804 München (DE); Wimmer, Viktor Josef, 88370 Seeon (DE)
(74) Vertreter: Eder, Christian

(56) Entgegenhaltungen:
- US-A- 4 772 093
- US-A- 5 573 494
- US-A- 5 575 752
- US-A1- 2004 225 191
- US-A1- 2005 075 538

## Beschreibung

Derartige Endoskope, wie beispielsweise in dem US-Patent 5573494 vorgeschlagen, finden in der Endoskopie zahlreiche Anwendung. Üblicherweise wird hierbei innerhalb eines vom proximalen bis zum distalen Endoskopende verlaufenden Instrumentierkanals eines bereits platzierten großen Endoskops ein Instrument, beispielsweise ein Katheter, ein kleines sogenanntes Babyendoskop mit einem geringeren Durchmesser etc., eingeführt, um eine endoskopische Untersuchung oder einen Eingriff vorzunehmen.

So wird beispielsweise bei der peroralen transpapillären Cholangioskopie zunächst ein flexibles Mutterendoskop (großes Duodenoskop) in der üblichen ERCP Technik an der Papilla major (im Weiteren Papille genannt) platziert. Zum Erreichen der Papille muss ein großer Teil des Duodenoskop-Schaftes in den Patienten (Schlund, Speiseröhre, Magen, Zwölffingerdarm)) eingeführt werden, weil das Endoskop entlang der zurückweichenden Kurvatur des Magens in das Duodenum vorgeschoben werden muss. Danach erfolgt ein Streckmanöver, bei dem ein großer Teil der Schaftlänge extrahiert wird, bis schließlich das Duodenoskop gestreckt an der Papille liegt. Nach der Streckung sind nur noch etwa 60 cm des vorderen Endoskopschaftes in den Patienten eingeführt. In dieser gestreckten Position werden sowohl ERCP als auch Mother-Baby-Cholangioskopie durchgeführt.

Die bisher übliche Standard-Mutter-Baby- (Mother-Baby)-Cholangioskopie erfordert ein (großes) Jumbo- bzw. Mutter-Duodenoskop und ein in das Mutter-Duodenoskop einführbares Baby-Cholangioskop. Das Babyendoskop wird hierbei über eine am Endoskopgehäuse bzw. Bedienteil befindliche Öffnung des Instrumentier- bzw. Arbeitskanals des Mutterendoskops in die Gallenwege eingeführt. Die Methode dient vorwiegend der Fragmentierung von großen Gallengangssteinen, beispielsweise mit einer Lasersonde und der Diagnostik unklarer Gallengangsbefunde.

Nachteiligerweise ist bei diesem bekannten Instrumentarium und Verfahren das Babyendoskop nur schwer oder kaum mehr manövrierbar. Darüber hinaus zeigt sich in der jahrelangen Praxis bei häufiger Anwendung eine hohe Reparaturanfälligkeit des Babyendoskops, welches häufig bereits nach wenigen Einsätzen defekt ist.

Der vorliegenden Erfindung liegt daher die Aufgabe zugrunde, diesen Stand der Technik zu verbessern und ein Duodenoskop sowie eine Anordnung aus einem Mutter- und Babyendoskop zu schaffen, welche diese Nachteile vermeiden und bei verbesserter Manövrierbarkeit eine geringere Reparaturanfälligkeit gewährleisten.

Diese Aufgabe wird erfindungsgemäß mit einem Duodenoskop mit den Merkmalen des Anspruchs 1 sowie einer Anordnung aus einem solchen Endoskop mit einem Babyendoskop mit den Merkmalen des Anspruchs 9 gelöst. Ein Endoskop mit einen vom proximalen Ende entfernten Eingangsöffnung ist aus Dokument US 2005/0075538 bekannt.

Da sich die erste Eingangsöffnung, im Folgenden Schaftport genannt, in einem vom proximalen Ende des Endoskops entfernten Bereich des Schaftes befindet, liegt der Schaftport nach einer erfolgten Streckung außerhalb des Patienten, beispielsweise einige Zentimeter vor seinem Mund, und kann jetzt als Zugang in den Instrumentierkanal bzw. Arbeitskanal des Endoskops benutzt werden. Dadurch kann ein erheblich verkürztes Babyendoskop verwendet werden, weil es über den Schaftport und nicht mehr über den sonst üblichen (proximalen) Eingang bzw. den proximalen Port des Instrumentierkanals am Bedienteil des Endoskops eingeführt wird. Statt Instrumenten, wie beispielsweise einem Babyendoskop, einem Katheter etc., welche eine Länge von beispielsweise ca. 200 cm bei einem Durchmesser von 3 bis 4 mm erfordern, um die Schaftlänge des Mutterendoskops zu überragen, können nun vorteilhafterweise wesentlich verkürzte Instrumente, beispielsweise ein Babyendoskop von einer Länge von nur 90 bis 140 cm, insbesondere 100 bis 120 cm verwendet werden.

Das verkürzte Instrument, insbesondere das verkürzte Babyendoskop ist besser manövrierbar, besser drehbar als herkömmliche längere Instrumente, wobei auch die Reinigung eines solchen kürzeren Instruments erleichtert wird. Zudem verbessert sich die Abwinkelbarkeit, wobei sich als Kostenvorteil auch die Reparaturanfälligkeit verringert.

Als verkürzte Instrumente kommen hierbei neben einem Babyendoskop (Cholangioskop, Pankreatikoskop) beispielsweise auch drehstabile Katheter vom Typ Angiografiekatheter in Frage, welche über die erste Eingangsöffnung bzw. den Schaftport in die Gallenwege eingeführt werden, um bisher schlecht oder gar nicht zugängliche periphere Gallengangsäste zu erreichen. Während sich über normale Duodenoskope eingeführte Angiografiekatheter infolge der großen Länge nicht mehr drehen lassen, ist dies nunmehr durch die infolge des Schaftports verkürzte Strecke bzw. deren verkürzte Ausbildung möglich. So lassen sich die verschiedenen Segmente des baumartigen biliären Systems, die sonst nur durch langes geduldiges "Stochern" bzw. ungezieltes Sondieren mit Führungsdrähten und Kathetern mehr oder weniger zufällig oder auch gar nicht erreicht werden können, gezielt sondieren.

Nach der Erfindung liegt der Schaftport im mittleren Bereich des Schaftes, insbesondere in einer Entfernung vom distalen Ende des Endoskops von 60 bis 70 cm. Hierdurch kann bei besonderen Untersuchungen, insbesondere der Cholangioskopie, gewährleistet werden dass die erste Öffnung außerhalb des Patienten, aber doch nahe seinem Mund zu liegen kommt. Ein einzuführendes Instrument bedarf daher nur einer Mindestlänge von beispielsweise 80 bis 140 cm, vorzugsweise 120 cm, um den nach der Platzierung in dem Patienten befindlichen Teil des Schaftes zu überragen.

In bevorzugter Ausgestaltung der Erfindung weist das Endoskop zusätzlich im Bereich des proximalen Endes bzw. am Endoskopgehäuse eine weitere Eingangsöffnung in Form eines üblichen proximalen Ports auf. Hierdurch ist das Endoskop zusätzlich als normales Endoskop, beispielsweise Duodenoskop anwendbar, so dass die Kosten für eine gesonderte Anschaffung eines solchen Endoskops eingespart werden können.

Hierbei können die erste und die weitere Eingangsöffnung bzw. der Schaftport und der proximale Port zu ein und demselben Arbeitskanal führen, wobei sich der Arbeitskanal hierbei bis zum proximalen Port erstreckt. Hierdurch kann vorteilhafterweise ein zusätzlicher separater Arbeitskanal vermieden werden, welcher sonst möglicherweise zu einer unerwünschten Erhöhung des Schaftdurchmessers des Endoskops führt.

In weiterer Ausgestaltung der Erfindung ist der Schaftport mit einer Abdeckung dichtend verschließbar ausgebildet. Dies kann beispielsweise durch eine Abdeckung in Form eines von außen ortsfest anbringbaren Deckels erfolgen, welcher vorzugsweise sowohl gegenüber dem Arbeitskanal als auch zum Endoskopinneren im aufgesetzten, beispielsweise verriegelten Zustand dichtet. Selbstverständlich sind auch andere Möglichkeiten denkbar, um die erste Eingangsöffnung bzw. den Schaftport vor allem während der Phase der Platzierung des Endoskops dichtend zu verschließen. Dies kann beispielsweise erreicht werden durch eine über den konventionellen Zugang am Bedienteil des Endoskops in den Endoskopkanal eingeführte Sonde, insbesondere Hohlsonde (um eine weiter Insufflation zu ermöglichen), die den Schaftport von innen (innere Abdeckung) dichtend schließt oder durch eine Verschlusshülse, deren Öffnung durch Drehen oder Verschieben gegenüber der Schaftport-Öffnung (äußere Abdeckung) geschlossen wird. Auch ist es denkbar einen dünnwandigen Gummi-, Kunststoffschlauch (beispielsweise des Typs "Fingerling" mit abgeschnittener Kuppe) zu verwenden, der auf das Duodenoskop aufgeschoben, über die Schaftportöffnung (äußere Abdeckung) abgerollt wird.

In weiterer Ausgestaltung der Erfindung kann an dem Schaftport ein Aufsatz mit einem Einführstutzen ortsfest und abnehmbar angeordnet werden, um das Einführen von Instrumenten in den Arbeitskanal zu erleichtern. Dieser Portaufsatz dichtet vorzugsweise die erste Eingangsöffnung bzw. den Schaftport ab, so dass eventuell unter Verwendung einer Abschlusskappe oder Dichtung keine Luft entweichen kann. Der Aufsatz kann hierbei derart an dem Schaftport bzw. in diesem Bereich des Schaftes angeordnet werden, so dass der Einführstutzen im Querschnittsprofil seitlich versetzt, beispielsweise um 90° bis 180°, auf der anderen Seite eines üblichen proximalen Ports zum Liegen kommt. Hierdurch kann vorteilhafterweise vermieden werden, dass sich Bedienpersonal und Arzt während einer Behandlung gegenseitig behindern, da nunmehr zum Hantieren bzw. Einführen und Bedienen eines Instruments die gegenüberliegende Seite (also beispielsweise links vom Bedienteil aus gesehen) des üblicherweise (in der Regel rechtsseitigen) proximalen Ports verwendet wird.

In bevorzugter Ausgestaltung der Erfindung ist der Bereich des Endoskopschaftes, in welchem sich der Schaftport befindet, als kurze, beispielsweise kleiner als 10 cm, insbesondere 4 bis 6 cm lange, starre (Schaft-)Hülse, beispielsweise in Form eines starren äußeren Schaftsegmentes und eines ebenfalls starren inneren Kanalsegmentes, ausgebildet. Hierdurch erhöht sich vorteilhafterweise trotz guter Flexibilität des gesamten Endoskopschaftes die Stabilität und Passgenauigkeit des Schaftports, so dass an dieser leichter eine Abdeckung in Form eines einsetzbaren Deckels oder ein Portaufsatz mit einem möglichst zum Arbeitskanal flachen Einführwinkel dichtend und ortsfest angeordnet werden kann.

Weitere vorteilhafte Ausgestaltungen der Erfindung ergeben sich aus den Unteransprüchcn

Die Erfindung wird nachfolgend anhand in der Zeichnung dargestellter Ausführungsbeispiele näher erläutert. In der Zeichnung zeigen:
Fig. 1a eine Seitenansicht eines Schaftports eines Endoskops nach der Erfindung mit Deckel und Arbeitskanal;
Fig. 1b eine Schnittansicht des Schaftports nach Fig. 1a entlang der Linie A-A;
Fig. 1c eine Schnittansicht des Schaftports nach Fig. 1a entlang der Linie B-B;
Fig. 2a eine Unteransicht eines Deckels;

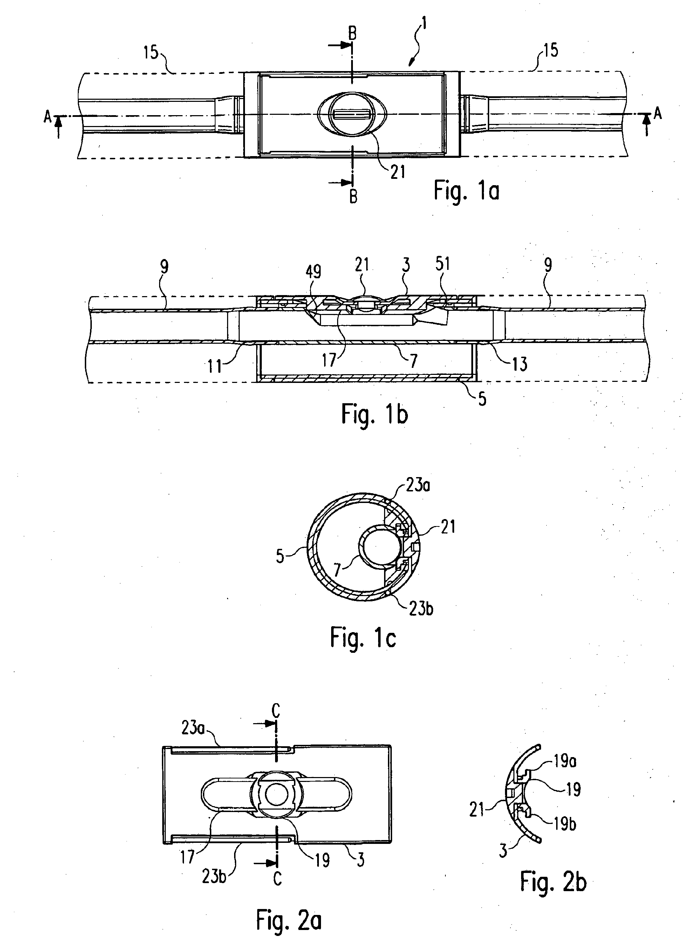

Fig. 2beine Schnittansicht des Deckels nach Fig. 2a entlang der Linie C-C;

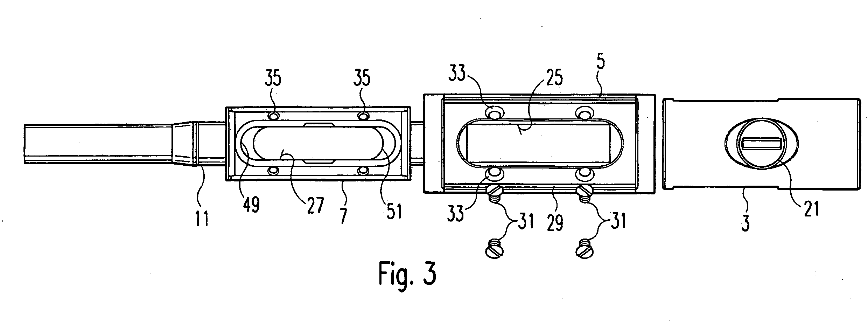

Fig. 3 eine Explosionsdarstellung eines Schaftports nach Fig. 1a:

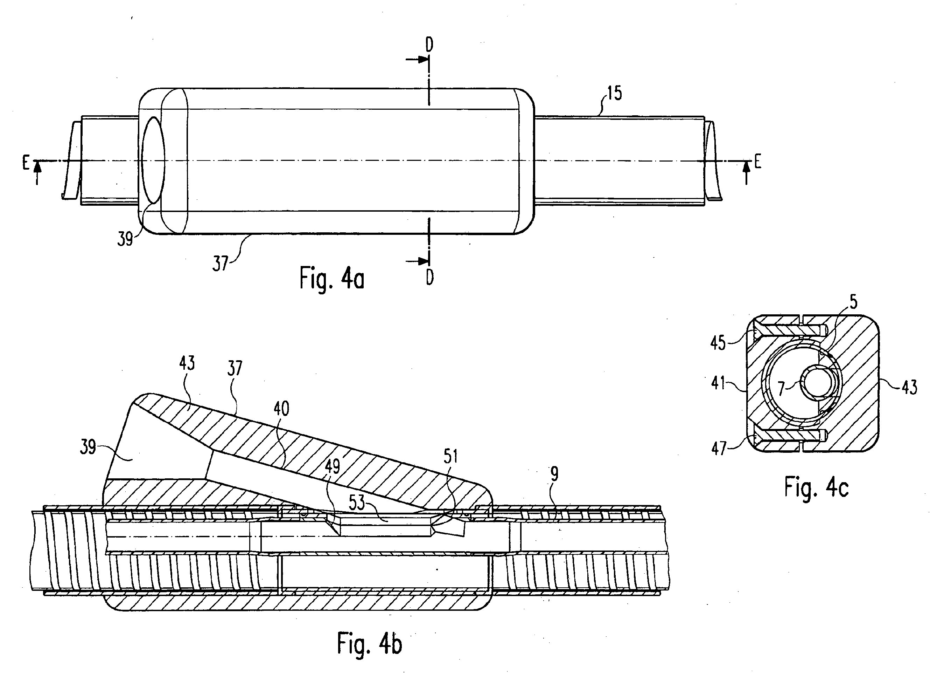

Fig. 4a eine Seitenansicht des Schaftports mit Portaufsatz;
Fig. 4b eine Schnittansicht des Schaftports nach Fig. 4a entlang der Linie E-E;
Fig. 4c eine Schnittansicht des Schaftports nach Fig. 4a entlang der Linie D-D;

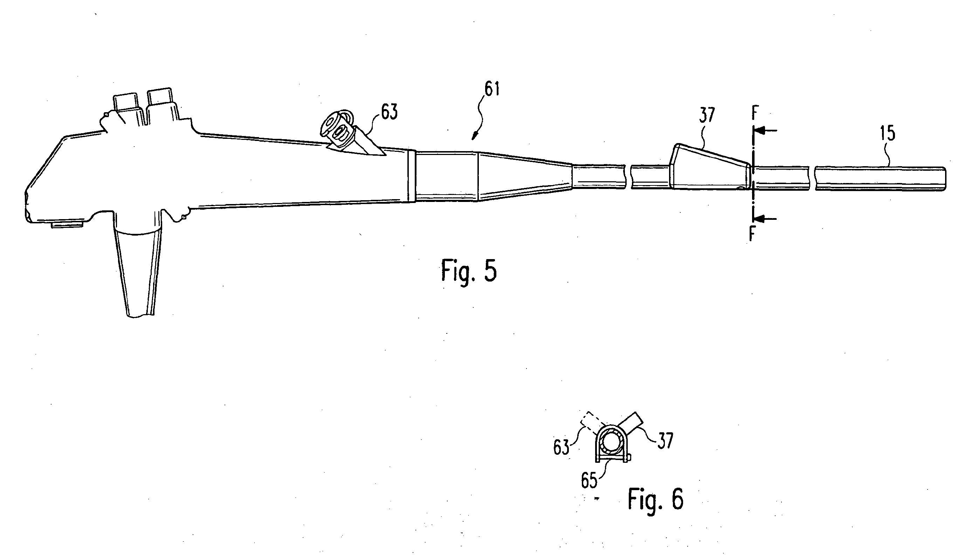

Fig. 5 eine Seitenansicht eines erfindungsgemäßen Endoskops und
Fig. 6 ein schematischer Querschnitt durch das Endoskop nach Fig. 5 entlang der Linie F-F

Die in Fig. 1a bis Fig. 1c dargestellte erste Eingangsöffnung bzw. der Schaftport 1 eines Endoskops ist mit einer (äußeren) Abdeckung in Form eines Deckels 3 dargestellt. Der Schaftport 1 umfasst ein Schaftsegment in Form einer äußeren starren Hülse 5 sowie ein Kanalsegment in Fonn einer im Durchmesser kleineren inneren starren Hülse 7, welche zur Durchleitung eines Arbeitskanals 9 des Endoskops dient und im Querschnitt (Fig. 1c) nicht konzentrisch, sondern nahe der Innenwandung des Schaftsegments 5 angeordnet ist.

Um eine in den Arbeitskanal 9 reichende Öffnung zu realisieren weisen sowohl Schaftscgment 5 als auch Kanalsegment 7 vorzugsweise zueinander fluchtende längliche Öffnungen 25, 27 (Fig. 3) auf Der auf diese Weise realisierte Zugang zum Arbeitskanal 9 kann wie in Fig. 1b dargestellt eine Einführhilfe in Form einer sich nach außen erweiternden Fase 49 aufweisen, um das Einführen eines Instruments in den Arbeitskanal 9 zu erleichtern. Zusätzlich kann sich an der gegenüberliegenden kurzen Seite der länglichen Schaftportöffnung eine sich nach innen erweiternde Schräge bzw. Einführhilfe 51 befinden, um das schräge Einführen eines Instruments von schräg links oben in den nach rechts in Richtung distales Ende führenden Arbeitskanal 9 zu erleichtern. Auf diese Weise kann vorteilhafterweise auch bei einem kurzen Schaftport 1 bzw. erste Eingangsöffnung von beispielsweise 15-30 mm ein leichtes Einführen eines Instruments gewährleistet werden.

Zum beidseitigen Anschluss an den als Teflonschlauch ausgebildeten Arbeitskanal 9 weist das Kanalsegment 7 zwei Anschlussstutzen 11 und 13 auf, auf welche die Enden des Arbeitskanals 9 aufgeschoben und eventuell zusätzlich, beispielsweise durch Verkleben, fixiert werden können.

Das Schaftsegment 5 ist im Endoskopschaft 15, welcher in Fig. 1a und Fig. 1b gestrichelt dargestellt ist, fest, beispielsweise durch Verkleben, fluchtend verbunden, so dass sich trotz der vorzugsweisen starren Ausbildung des gesamten Schaftports 1 an der Außenfläche des Endoskopschaftes 15 keine unerwünschten Angriffsstellen, wie Stufen, Kanten etc. als Gleithindernis bilden.

Wie aus Fig. 1b ersichtlich, schließt der dünne Deckel 3 (von beispielsweise 0,3 mm Dicke) den Schaftport 1 bzw. die erste Eingangsöffnung überdeckend ab, wobei ein hervorspringender Bereich 17 an der Unterseite des Deckels 3 in die längliche Öffnung 25 des Schaftsegments 5 und eventuell sogar in die hiermit fluchtende Öffnung 27 des Kanalsegments 7 vorzugsweise passgenau hineinragt. Hierdurch kann auf einfache Weise gewährleistet werden, dass der Deckel 3 ortsfest am Schaftport 1 befestigt werden kann. Hierzu wird ein Betätigungselement, beispielsweise in Form einer geschlitzten Schraube 21 einer Verriegelungseinrichtung betätigt, wobei die Verriegelungseinrichtung beispielsweise aus einem Verriegelungselement 19 besteht. Dieses Verriegelungselement 19 ist an der Unterseite der Schraube 21 ovalförmig ausgebildet, wobei nur im verriegelten Zustand gegenüberliegende Erweiterungen 19a und 19b der im wesentlichen ovalen Scheibe 19 in entsprechende Ausnehmungen des Kanalsegments 7 eingreifen. Selbstverständlich ist es auch denkbar, den Deckel 3 aufklippbar auszubilden, wobei er im Profil beispielsweise hierzu als eine über den Schaftumfangshälfte ragende bzw, umgreifende Halbschale ausgebildet ist. Der hervorspringender Bereich 17 kann in jedem Fall zusätzlich zum Kanalinneren fluchtend ausgebildet sein, so dass sich im verschlossenen Zustand vorteilhafterweise für ein über den proximalen Port 63 eingeführtes Instrument keine unerwünschten Widerstände ergeben.

Wie aus Fig. 3 ersichtlich, ist der Schaftport 1 aus einem Kanalsegment 7 und dem Schaftsegment 5 derart aufgebaut, dass, wie aus Fig. 1c ersichtlich, das Kanalsegment mit seiner Öffnung 27 an der Innenseite des Schaftsegments 5 im Bereich der Öffnung 25 angeordnet ist. Zur einfachen Befestigung kann das Kanalsegment 7 im Querschnitt eine an der Außenfläche halbrunde Erweiterung aufweisen, in welcher Gewindebohrungen 35 vorgesehen sind. Im zusammengebauten Zustand liegen diese Bohrungen 35 fluchtend mit Bohrungen 33 im Schaftsegment 5, so dass das Kanalsegment 7 am Schaftsegment 5 mittels Schrauben 31 fixiert werden kann. Auf diese Weise gehen die Öffnung 25 des Schaftsegments 5 mit ihren Schrägen 49 und 51 und die Öffnung 27 des Kanalsegments 7 fluchtend ineinander über.

Zur Gewährleistung der Dichtigkeit zum Endoskopschaftinneren ist zwischen der Außenfläche des Kanalsegments 7 eine in der Zeichnung nicht näher dargestellte, um die Öffnung 27 umlaufende Dichtung vorgesehen, welche an der Innenseite des Schaftsegments 5 dichtend anliegt. Weiterhin ist an der Außenfläche des Schaftsegments 5 eine um die Öffnung 25 umlaufende Dichtung 29 im Bereich der Unterseite des Deckels 3 angeordnet, so dass der Deckel 3 im verriegelten Zustand ebenfalls dichtend abschließt. Zur Erhöhung des Gegendrucks der Dichtung 29 im Schließzustand können zusätzlich an beiden Längsseiten des Deckels 3 (oder der entsprechenden komplementären Ausnehmung des Schaftports 1) ein oder mehrere längliche Federelemente 23a und 23b angeordnet sein, welche derart vorgebogen sind, dass sie sich im schließenden Zustand länglich erstrecken und ihre Federkraft gegen das Aufsetzen des Deckels 3 wirkt.

In Fig. 4a ist dargestellt, wie ein Portaufsatz 37, welcher beispielsweise als Frästeil oder Spritzgussteil gefertigt ist, auf den Schaftport 1 aufgesetzt wird, um durch eine Öffnung 39 das Einführen eines Instruments in distale Richtung (in der Zeichnung nach rechts) in den Arbeitskanal 9 zu ermöglichen. Der Portaufsatz 37 wird hierbei durch die seitliche Öffnung 25, 27 auf den Schaftport 1 des Mutterendoskop-Schaftes 15 aufgesteckt. Zwischen Schaftport 1 und Portaufsatz 37 kann zudem eine in der Zeichnung nicht näher dargestellte Dichtung ("O"-Ring, Gummiplatte etc.) vorgesehen sein, um ein Entweichen von Luft zwischen Portaufsatz 37 und Schaftport 1 zu verhindern.

Gemäß Fig. 4c ist der Portaufsatz 37 aus einer Unterschale 41 und einer Oberschale 43, in welcher sich die trichterförmig nach außen erweiternde Öffnung 39 und sich gerade anschließenden Einführstutzen 40 befinden, aufgebaut. Zum Aufsetzen auf den Schaftport 1 können, wie dargestellt zwei Befestigungselemente 45 und 47, beispielsweise in Form von Verriegelungsstiften oder Schrauben angeordnet sein, wobei selbstverständlich auch ein Gelenk an einer Seite und eine an der gegenüberliegenden Seite angeordnete Verriegelung, beispielsweise in Form eines Hakens, denkbar ist.

Wie in Fig. 4b dargestellt führt der gerade rohrförmig ausgebildete Einführstutzen 40 von links oben nach schräg rechts unten in einem flachen Winkel in den Arbeitskanal 9 in distale Richtung (in der Zeichnung rechte Seite) hinein, wobei diese Führung durch an den kurzen Seiten der länglichen Öffnungen 25 und 27 ineinander übergehende Schrägen 49 und 51 unterstützt wird. Der Einführstutzen 40 besitzt hierbei einen gegenüber dem Innendurchmesser des Arbeitkanals (von beispielsweise 4,2 mm) kleiner oder gleichen Innendurchmesser. Zudem ragt der Portaufsatz 37 an dieser Stelle mit seinem entsprechend der Öffnung 25 ausgebildeten Material 53 hinein, so dass durch dieses ineinanderpassende Eingreifen eine orts- und drehfeste Anordnung und Zentrierung des Portaufsatzes 37 am Schaftport 1 gewährleistet ist. Auf diese Weise kann vorteilhafterweise auf das Vorsehen von für eine ortsfeste Verbindung sonst notwendigen Ausnehmungen oder Vorsprüngen an der Außenfläche des Schaftes 15 und/oder des Schaftports 1 verzichtet werden, welche den Reibungs- bzw. Gleitwiderstand bei einer Behandlung unerwünscht erhöhen. Durch die Einführhilfen 49, 51 wird selbst bei erwünschter kurzer Länge des Schaftports 1 bzw. der Schaft- und Kanalsegmente 5 und 7 von 40 bis 60 mm und einer durch die Öffnungen 25, 27 realisierten kurzen Schaftportöffnung von nur 15 bis 30 mm Länge ein möglichst flacher bzw. spitzer Winkel (zum proximalen Ende hin gesehen) zwischen der Längsachse des Einführstutzens und der Längsachse des Arbeitskanals 9 bzw. des Endoskopschafts 15 von beispielsweise 10 bis 40 Grad, vorzugsweise von 10 bis 20 Grad, ermöglicht. Zudem kann der Einführstutzen 40 bzw. die Öffnung 39 mit einer in der Zeichnung nicht näher dargestellten Dichtungskappe (auch für das dichtende Einführen von Instrumenten) verschlossen werden.

Aus Fig. 5 und Fig. 6 wird ersichtlich, auf welche Art und in welcher Position der Schaftport 1 bzw. der Portaufsatz 37 bei einem hier dargestellten Mutter- bzw. Mother-Duodenoskop 61 angeordnet ist. Der 40 bis 60 mm lange, in den Schaft 15 integrierte Schaftport 1 befindet sich hierbei in Längsrichtung etwa zwischen 60 und 70 cm Entfernung vom distalen Kopfende des Endoskops 61. Die längliche Öffnung des Schaftports 1 besitzt beispielsweise für das Einführen eines im Durchmesser 3,5 mm dicken Babyendoskops eine Länge von etwa 15 bis 30 mm. Durch den Portaufsatz 37 wird ein möglichst spitzer Einführwinkel, beispielsweise von etwa 10 bis 30 Grad zur Längsachse des Endoskops 61 ermöglicht.

Aus der stark schematisierten Querschnittsansicht nach Fig. 6 wird ersichtlich, dass der Schaftport 1 bzw. Portaufsatz 37 mit seiner Öffnung 39 in Längsrichtung am proximalen Ende bzw. vom Bedienteil des Endoskops aus gesehen linksseitig angeordnet ist, wohingegen ein herkömmlicher, proximaler Port 63 üblicherweise am Bedienteil rechtsseitig angeordnet ist. Durch diese gegenüberliegende Anordnung, beispielsweise von 90 bis 180 Grad, insbesondere symmetrisch zu einer Mittelachse vorgesehenen Anordnung ist es vorteilhafterweise möglich, dass während einer Behandlung drei Personen gleichzeitig, also am Bedienteil, rechts davon an einem Instrument, welches durch den proximalen Port 63 eingeführt ist und links davon an einem Instrument, welches durch den Port 37 eingeführt wird, arbeiten können, ohne sich gegenseitig zu behindern.

In Fig. 6 ist zudem stark vereinfacht angedeutet, dass der Portaufsatz statt zweischalig beispielsweise auch im Querschnittsprofil u-förmig ausgebildet sein kann. Zudem ist in Fig. 6 eine anderer Art der Befestigung, nämlich beispielsweise mittels einer in die Enden der U-förmigen Schenkel einsetzbare Schraube oder Achse mit einem Excenter, welcher bei Betätigung durch Drehung den Portaufsatz an den Schaft bzw. den Schaftport festklemmt, angedeutet.

Im Folgenden wird die Funktionsweise des erfindungsgemäßen Endoskops anhand einer endoskopisch retrograden Cholangiopankreatographie (ERCP) erläutert.

Während der Phase der Platzierung des Mutter-Duodenoskops 61 über Schlund, Speiseröhre und Magen ins Duodenum muss der Schaftport 1 verschlossen sein, da sich hierbei etwa 100 bis zu 110 cm des Endoskopschafts und damit der Schaftport selbst im Inneren eines Patienten befinden. Dies kann beispielsweise erreicht werden durch einen, wie vorstehend erläutert, in die Schaftportöffnung einsetzbaren Deckel 3. Selbstverständlich sind aber auch andere Formen von inneren oder äußeren Abdeckungen, wie beispielsweise eine über den konventionellen proximalen Zugang bzw. Port 63 am Bedienteil des Endoskops in den Endoskopkanal 9 eingeführte Hohlsonde, die den Schaftport 1 von innen dichtend schließt, eine Verschlusshülse, deren Öffnung durch Drehen oder Verschieben gegenüber der Schaftportöffnung geschlossen wird oder ein dünnwandiger Gummi-, Kunststoffschlauch (Typ Fingerling mit abgeschnittener Kuppe) der auf das Duodenoskop aufgeschoben, über die Schaftportöffnung angerollt wird, denkbar.

Nach der Platzierung kommt der Schaftport 1 außerhalb des Patienten zu liegen, da infolge der Streckung nur eine Schaftlänge von weniger als 60 cm im Patienten verbleibt. Nach Entfernen der Abdeckung und Befestigung des Portaufsatzes 37 kann der Schaftport 1 dann als Zugang für Instrumente genutzt werden. Hierzu wird der Portaufsatz 37 gegen das jeweilige Instrument, beispielsweise ein eingeführtes Babyendoskop abgedichtet (beispielsweise mittels einer entsprechenden Dichtung im Portaufsatz 37 bzw. dessen Öffnung, um zu verhindern, dass während des Einführens bis hin zum Duodenum eingeblasene Luft entweicht und somit durch Zusammenfallen des Lumens des Duodenums die endoskopische Sicht verschlechtert. Selbstverständlich ist es auch denkbar Instrumente direkt in den Schaftport 1 ohne Portaufsatz 37 einzuführen, wobei hierzu vorzugsweise eine stärkere Luftzufuhr erfolgen muss, um die möglicherweise über den Schaftport 1 entweichende Luft zu kompensieren.

Der Portaufsatz 37 wird über den Schaftport 1 in den Instrumentierkanal bzw. Arbeitskanal 9 eingeführt und dichtet diesen ab. Am äußeren Ende kann zudem ein seitlicher Stutzen für die Luftinsufflation angebracht sein. Dieser Stutzen kann eventuell an die normale Luftzufuhr angeschlossen sein. Selbstverständlich ist es auch denkbar den Portaufsatz 37 im Prinzip als einen sehr dünnwandigen (Teflon etc.) Schlauch auszubilden, welcher über den Schaftport 1 in den Arbeitskanal 9 im Wesentlichen dichtend eingeschoben wird. Ein solcher Portaufsatz 37 sollte dann vorzugsweise eine dichtende Kappe sowie eventuell zur Insufflation hilfreichen seitlichen Luftstutzen aufweisen.

Die Fixierung des Portaufsatzes 37 auf dem Schaft 15 kann durch verschiedene Möglichkeiten, wie beispielsweise einen Excenter (Schraube, Hebel o. ä.) mit dem der Portaufsatz 37 geklemmt wird, erfolgen. Der Portaufsatz 37 ist vorzugsweise mit Zentriermittel, wie beispielsweise durch den vorstehend erläuterten Vorsprung, Zentrierungsstiften o. ä., ausgestattet und derart auf dem Schaftport 1 fixiert, dass der Einführstutzen 40 optimal in den Instrumentierkanal 9 mündet und somit das Babyendoskop optimal mit möglichst geringer Reibung in das Mutterendoskop 61 eingeführt werden kann.

Selbstverständlich ist es auch denkbar den Portaufsatz 37 ebenso aufklippbar auszubilden, wie es vorstehend anhand der klippbaren Variante des Deckels beschrieben wurde.

Ein derartiges Babyendoskop besitzt beispielsweise einen Außendurchmesser von etwa 3,5 mm und weist somit zum Instrumentierkanal (Durchmesser etwa 4 mm) des Mutterendoskops etwa 0,5 mm Spiel auf (z.B. für die Insufflation). Die Außenhaut des Babyendoskops ist möglichst gleitfähig gestaltet (Teflon etc.), um einen geringen Gleitwiderstand zu bieten. Bei Abwicklung in nur einer Ebene (auf und ab), ist das Babyendoskop vorzugsweise möglichst rotationsstabil, damit die fehlenden Abwinklungsebenen durch Rotation erreicht werden können. Das Babyendoskop weist einen Instrumentierkanal von mindestens 1 mm (vorzugsweise mindestens 1,4 mm) auf, so dass es für elektrohydraulische Sonden, Biopsiezangen, Steinkörbchen, etc. geeignet ist. Durch den Schaftport 37 ist eine gute Abwinkelung auch nach Einführen des Babyendoskops gewährleistet, so dass allein mit der guten Abwinkelbarkeit das Instrument seine klinische Relevanz erreicht wird.

Hervorragend anwendbar ist bei der Cholangioskopie mit einem verkürztem Baby die elektrohydraulische Lithotripsie. Die in der Länge an das Babyendoskop angepasste Sonde wird hierbei mit Spiel durch den Instrumentierkanal des Babyendoskops eingeführt. Bei eingeführter EHL-Sonde ist vorzugsweise eine ausreichende Perfusion mit der für die EHL erforderliche Flüssigkeit möglich.

Als weitere Anwendung sind aber auch drehstabile Katheter von ca. 120 cm Länge (beispielsweise Katheter Typ Angiografie) mit schräger Nase zum selektiven Sondieren der Gallenwege denkbar.

Weist ein Endoskop, insbesondere Duodenoskop zusätzlich zu dem erfindungsgemäßen Schaftport einen üblichen proximalen Port auf, kann das Endoskop im Prinzip auch als Routine-Endooskop, insbesondere Duodenoskop, beispielsweise für die konventionelle ERCP, eingesetzt werden, so dass nicht zwei Duodenoskope für den ERCP-Betrieb erforderlich sind. Hierdurch können vorteilhafterweise (besonders in kleineren Kliniken mit beschränktem Budget) Kosten gespart werden.

## Patentansprüche

1. Duodenoskop für die Mutter-Baby-Cholangioskopie, mit einem in oder an einem Schaft (15) des Endoskops (61) verlaufenden Arbeitskanal (9), welcher distal am Endoskopkopf eine Ausgangsöffnung aufweist,
**dadurch gekennzeichnet, dass**
der Arbeitskanal (9) wenigstens eine erste Eingangsöffnung (1) aufweist, welche in einem vom proximalen Ende des Endoskops (61) entfernten Bereich des Schaftes (15) liegt,
der Bereich des Endoskopschaftes (15), in welchem sich die erste Eingangsöffnung (1) befindet, als starre Hülse (5, 7) ausgebildet ist und
die starre Hülse (5) im Endoskopschaft (15) fluchtend verbunden ist, so dass sich an der Außenfläche des Endoskopschaftes (15) keine unerwünschten Angriffsstellen als Gleithindemis bilden.

2. Endoskop nach Anspruch 1 , **dadurch gekennzeichnet, dass** das Endoskop (61) zusätzlich im Bereich des proximalen Endes bzw. am Endoskopgehäuse eine weitere Eingangsöffnung (63) aufweist.

3. Endoskop nach Anspruch 2, **dadurch gekennzeichnet, dass** die erste und die weitere Eingangsöffnung (1, 63) zu ein und demselben Arbeitskanal (9) führen, wobei sich der Arbeitskanal (9) hierbei bis wenigstens zur weiteren Eingangsöffnung (63) erstreckt.

4. Endoskop nach einem der vorhergehenden Ansprüche, **dadurch gekennzeichnet, dass** die erste Eingangsöffnung (1) mit einer ortsfesten Abdeckung (3) dichtend verschließbar ausgebildet ist.

5. Endoskop nach Anspruch 4, **dadurch gekennzeichnet, dass**, die Abdeckung (3) an ihrer Unterseite einen hervorspringenden Bereich (17) aufweist, welcher in die Öffnung (27) des Kanalsegments (7) hineinragt, wobei der hervorspringende Bereich (17) zum Kanalinneren fluchtend ausgebildet ist, so dass sich im verschlossenen Zustand für ein über einen proximalen Port (63) eingeführtes Instrument keine unerwünschten Widerstände ergeben.

6. Endoskop nach einem der vorhergehenden Ansprüche, **dadurch gekennzeichnet, dass** an der ersten Eingangsöffnung (1) ein Aufsatz (37) mit einem Einführstutzen (40) ortsfest und abnehmbar anordenbar ist, um das Einführen von Instrumenten in den Arbeitskanal (9) zu erleichtern.

7. Endoskop nach Anspruch 6, **dadurch gekennzeichnet, dass** im aufgesetzten Zustand des Aufsatzes (37) zwischen der Längsachse des Einführstutzens (40) und der Längsachse des Arbeitskanals (9) ein in Richtung des proximalen Endes des Endoskops gesehener spitzer Winkel von 10 bis 40 Grad besteht.

8. Endoskop nach Anspruch 6 oder 7, **dadurch gekennzeichnet, dass** im aufgesetzten Zustand der Aufsatz (37) im Profil seitlich versetzt auf der gegenüberliegenden Seite einer proximalen weiteren Eingangsöffnung (63) angeordnet ist.

9. Anordnung aus einem Endoskop nach einem der Ansprüche 1 bis 8 und einem Baby-Endoskop, welches in den Arbeitskanal (9) eines Endoskops (61) nach einem der Ansprüche 1 bis 8 eingeführt werden kann und eine Länge von 80 bis 140 cm, vorzugsweise 120 cm, aufweist, um eine Spiegelung der Gallenwege (Cholangioskopie) durchzuführen.

## Claims

1. A duodenoscope for mother-baby cholangioscopy having a working channel (9) which runs in or on a shaft (15) of the endoscope (61) and has an outlet opening distally at the endoscope head,
**characterised in that**
the working channel (9) has at least one first inlet opening (1) which lies in a region of the shaft (15) that is remote from the proximal end of the endoscope (61),
the region of the endoscope shaft (15), in which the first inlet opening (1) is located, is formed as a rigid sleeve (5, 7), and
the rigid sleeve (5) is connected in alignment in the endoscope shaft (15) so that no undesirable application points form as a hindrance to sliding on the outer face of the endoscope shaft (15).

2. An endoscope according to claim 1, **characterised in that** the endoscope (61) additionally has a further inlet opening (63) in the region of the proximal end or on the endoscope housing.

3. An endoscope according to claim 2, **characterised in that** the first and the further inlet opening (1, 63) lead to one and the same working channel (9), wherein the working channel (9) in this case extends as far as at least the further inlet opening (63).

4. An endoscope according to one of the preceding claims, **characterised in that** the first inlet opening (1) is formed so that it can be closed with a fixed cover (3) in a sealing manner.

5. An endoscope according to claim 4, **characterised in that** the cover (3) has on its underside a protruding region (17) which projects into the opening (27) of the channel segment (7), wherein the protruding region (17) is formed so that it is in alignment with the channel interior so that in the closed state no undesirable instances of resistance result for an instrument inserted by way of a proximal port (63).

6. An endoscope according to one of the preceding claims, **characterised in that** an attachment (37) with an insertion connection piece (40) can be arranged at the first inlet opening (1) in a fixed and removable manner in order to facilitate the insertion of instruments into the working channel (9).

7. An endoscope according to claim 6, **characterised in that** in the attached state of the attachment (37) an acute angle of 10 to 40 degrees, when viewed in the direction of the proximal end of the endoscope, exists between the longitudinal axis of the insertion connection piece (40) and the longitudinal axis of the working channel (9).

8. An endoscope according to claim 6 or 7, **characterised in that** in the attached state the attachment (37) is arranged in such a way that it is offset laterally in profile on the opposite side of a proximal further inlet opening (63).

9. An arrangement consisting of an endoscope according to one of claims 1 to 8 and a baby endoscope which can be inserted into the working channel (9) of an endoscope (61) according to one of claims 1 to 8 and has a length of 80 to 140 cm, preferably 120 cm, in order to effect mirroring of the biliary ducts (cholangioscopy).

## Revendications

1. Duodénoscope pour cholangioscopie mère-enfant, avec un canal de travail (9) s'étendant dans ou sur une tige (15) de l'endoscope (61), qui comprend, du côté distal, sur la tête de l'endoscope, une ouverture de sortie,
**caractérisé en ce que**
le canal de travail (9) comprend au moins une première ouverture d'entrée (1), qui se trouve dans une zone de la tige (15) éloignée de l'extrémité proximale de l'endoscope (61),
la zone de la tige de l'endoscope (15), dans laquelle se trouve la première ouverture d'entrée (1), est conçue comme une gaine rigide (5, 7) et
la gaine rigide (5) est reliée de manière alignée dans la tige de l'endoscope (15), de façon à ce qu'aucun point d'attaque indésirable ne se forme à la surface externe de la tige de l'endoscope (15) et empêche tout glissement.

2. Endoscope selon la revendication 1, **caractérisé en ce que** l'endoscope (61) comprend, en outre, dans la zone de l'extrémité proximale ou sur le boîtier de l'endoscope, une ouverture d'entrée supplémentaire (63).

3. Endoscope selon la revendication 2, **caractérisé en ce que** la première ouverture d'entrée et l'ouverture d'entrée supplémentaire (1, 63) mènent au même canal de travail (9), le canal de travail (9) s'étendant au moins jusqu'à l'ouverture d'entrée supplémentaire (63).

4. Endoscope selon l'une des revendications précédentes, **caractérisé en ce que** la première ouverture d'entrée (1) peut être fermée de manière étanche avec un couvercle fixe (3).

5. Endoscope selon la revendication 4, **caractérisé en ce que** le couvercle (3) comprend, sur son côté inférieur, une zone saillante (17) qui dépasse dans l'ouverture (27) du segment de canal (7), la zone saillante (17) étant alignée avec l'intérieur du canal, de façon à ce que, dans l'état fermé, aucune résistance indésirable n'apparaisse pour un instrument introduit par un orifice proximal (63).

6. Endoscope selon l'une des revendications précédentes, **caractérisé en ce que**, au niveau de la première ouverture d'entrée (1), un élément (37), avec un embout d'introduction (40), peut être disposé de manière fixe et amovible afin de faciliter l'introduction d'instruments dans le canal de travail (9).

7. Endoscope selon la revendication 6, **caractérisé en ce que**, dans l'état monté de l'élément (37), entre l'axe longitudinal de l'embout d'introduction (40) et l'axe longitudinal du canal de travail (9), il existe un angle aigu de 10 à 40 degrés, vu en direction de l'extrémité proximale de l'endoscope.

8. Endoscope selon la revendication 6 ou 7, **caractérisé en ce que**, dans l'état monté, l'élément (37) est disposé de manière décalée latéralement dans le profil sur le côté opposé d'une ouverture d'entrée supplémentaire proximale (63).

9. Dispositif constitué d'un endoscope selon l'une des revendications 1 à 8 et un endoscope pour bébé, qui peut être introduit dans le canal de travail (9) d'un endoscope (61) selon l'une des revendications 1 à 8 et qui présente une longueur de 80 à 140 cm, de préférence de 120 cm, afin de réaliser une image miroir des voies biliaires (cholangioscopie).
